(19) Europäisches Patentamt
European Patent Office
Office européen des brevets

(11) Veröffentlichungsnummer: **0 161 600**
**A2**

(12) # EUROPÄISCHE PATENTANMELDUNG

(21) Anmeldenummer: **85105480.9**

(22) Anmeldetag: **06.05.85**

(51) Int. Cl.[4]: **C 07 C 85/26**

(30) Priorität: **16.05.84 DE 3418090**

(43) Veröffentlichungstag der Anmeldung: **21.11.85 Patentblatt 85/47**

(84) Benannte Vertragsstaaten: **AT BE CH DE FR GB IT LI NL SE**

(71) Anmelder: **BAYER AG, Konzernverwaltung RP Patentabteilung, D-5090 Leverkusen 1 Bayerwerk (DE)**

(72) Erfinder: **Knöfel, Hartmut, Dr., Dülmener Weg 21, D-5068 Odenthal (DE)**
Erfinder: **Brockelt, Michael, Neuenhauser Weg 1, D-5060 Bergisch-Gladbach 2 (DE)**
Erfinder: **Hammen, Günter, Dr., Bunzlauer Weg 13, D-4049 Rommerskirchen (DE)**

(54) **Verfahren zur Isolierung von aromatischen Monoaminen aus Amingemischen.**

(57) Ein neues Verfahren zur Isolierung von aromatischen Monoaminen in reiner oder angereicherter Form als Lösung in einem hydrophoben Lösungsmittel oder, in zumindest teilweise protonierter Form, als wäßrige Lösung aus einem, mindestens zwei unterschiedliche aromatische Monoamine enthaltenden Amingemisch, bei welchem man das Ausgangsgemisch in einem ersten Arbeitsgang in einem zweiphasigen System bestehend aus einer hydrophoben Lösungsmittelphase und einer wäßrigen sauren Phase unter Durchmischung der Phasen verteilt und die hierbei anfallenden Phasen nach ihrer Trennung isoliert und gegebenenfalls in einem zweiten Arbeitsgang die Lösungsmittelphase erneut mit einer wäßrigen Phase oder die wäßrige Phase erneut mit einer Lösungsmittelphase extrahiert und die hierbei anfallenden Phasen aufarbeitet.

EP 0 161 600 A2

0161600

BAYER AKTIENGESELLSCHAFT 5090 Leverkusen, Bayerwerk
Konzernverwaltung RP Wr/by-c
Patentabteilung

# Verfahren zur Isolierung von aromatischen Monoaminen aus Amingemischen

Die Erfindung betrifft ein neues Verfahren zur Isolierung von aromatischen Monoaminen in reiner oder angereicherter Form aus mindestens zwei aromatische Monoamine enthaltenden Amingemischen durch flüssig-flüssig-Extraktion unter Verwendung eines zweiphasigen Systems bestehend aus einer sauren wäßrigen und einer organischen Phase.

Aromatische Monoamine sind u.a. wichtige Zwischenprodukte zur Synthese von Farbstoffen, Stabilisatoren, Pflanzenschutzmitteln und Pharmazeutika; allerdings fallen diese Amine bei der Herstellung normalerweise nicht in einer Reinheit an, die eine direkte Weiterverarbeitung erlauben würde, es sind vielmehr aufwendige Reinigungsoperationen notwendig, um z.B. isomere Verbindungen zu trennen oder bei nicht vollständig verlaufenden Umsetzungen zu den reinen aromatischen Monoaminen zu gelangen.

Die Lösung dieser Trennprobleme gemäß dem Stand der Technik ist dabei oft umständlich, aufwendig und in ihrer Effektivität beschränkt. So können z.B. o-, m- und p-Toluidin nicht mehr ausschließlich durch Rektifikation in die reinen Isomeren getrennt werden (Ullmanns Encyclopädie der technischen Chemie, 4. Auflage, Band 23, Seite 294). Um zu den reinen Verbindungen zu gelangen, wird auf der Stufe der Nitrotoluole zunächst o-Nitrotoluol abdestilliert, aus dem im Sumpf verbleibenden Gemisch aus m- und p-Nitrotoluol können dann durch weitere Destillations- und Kristallisationsschritte die jeweiligen Isomeren in reiner Form erhalten werden. Durch Reduktion der reinen Nitrotoluole erhält man schließlich die isomeren Toluidine als Reinprodukte (Ullmanns Encyclopädie der technischen Chemie, 4. Auflage, Band 17, Seite 390 ff).

Die Trennung der Isomeren auf der Nitrostufe durch Kombination der Verfahren Destillation und Kristallisation wird beispielsweise auch bei der Herstellung der Chloraniline oder Dichloraniline angewendet.

Ein weiteres Beispiel für eine nach dem Stand der Technik aufwendige Amintrennung ist die Herstellung von N-Methylanilin (Kp bei 1013 mbar 195°C) bzw. N,N-Dimethylanilin (Kp bei 1013 mbar 193°C) aus Anilin und Methanol in Gegenwart saurer Katalysatoren. N-Methyl und N,N-Dimethylanilin werden durch Vakuumdestillation (Kolonne mit 80 theoretischen Böden, hohes Rücklaufverhältnis) getrennt, eine Feinreinigung von N-Methylanilin erfordert die zusätzlichen Stufen der Acetylierung und der anschließenden Säurespaltung. Bei der Synthese von N,N-Dimethylanilin erhält man ein 92 bis 95 %iges Rohprodukt, aus dem N-Methylanilin und Anilin durch Umsetzung mit Phthalsäureanhydrid abgetrennt werden; erst dann ist eine Reindarstellung durch Destillation möglich (Ullmanns Encyclopädie der technischen Chemie, 4. Auflage, Band 7, Seite 572).

In der Literatur finden sich zwar bereits Hinweise, daß die Auftrennung von Gemischen isomerer bzw. homologer basischer Verbindungen durch selektive Extraktion möglich ist, jedoch vermittelt keine der einschlägigen Literaturstellen eine großtechnisch nacharbeitbare Lehre zum technischen Handeln bezüglich des Problems der Auftrennung von isomeren bzw. homologen aromatischen Monoaminen. So beschreibt beispielsweise W.N. Axe in J. Amer. Chem. Soc. Band 61, Seite 1017 ff. die Trennung von isomeren alkylsubstituierten Chinolinen nach einem extraktiven Verfahren; aus der Veröffentlichung ist aber nicht zu entnehmen, daß das Verfahren auch auf die Trennung von aromatischen Monoaminen anwendbar ist. Spryskov (Zhur. Priklad. Khim., 21, (1948), Seiten 156 ff., siehe auch Chemical Abstracts (1951), 1530 a-d) befaßt sich mit der Trennung von bestimmten Xylidinen nach einer extraktiven Methode, ohne allerdings das Verfahren genau zu beschreiben und die erreichten Reinheitsgrade

anzugeben. Die DE-OS 2 311 572 befaßt sich mit der Trennung von Aminoanthrachinonen mit Hilfe eines zweiphasigen Systems bestehend aus einer wäßrigen sauren Phase und einer hydrophoben organischen Phase bei 100 bis 160°C ohne Hinweis auf die erfindungsgemäß aufgefundene Möglichkeit, aromatische Monoamine nach einem speziellen Extraktionsverfahren bei vergleichsweise wesentlich tieferen Temperaturen aufzutrennen. C. Golumbic beschreibt schließlich in Anal. Chem. 24, 1849-50 (1952) ein Trennverfahren u.a. für isomere Toluidine in einem zweiphasigen System bestehend aus einem hydrophoben Lösungsmittel und einer wäßrigen Pufferlösung, deren Zusammensetzung nicht genau angegeben wird. Das kurze wissenschaftliche Referat beinhaltet keinerlei Lehre zum technischen Handeln, insbesondere keine Lehre wie aromatische Monoamine großtechnisch voneinander getrennt werden könnten.

Mit dem nachstehend näher beschriebenen erfindungsgemäßen Verfahren wird nunmehr ein großtechnisch gangbarer Weg zur wirkungsvollen Trennung von isomeren bzw. homologen aromatischen Monoaminen eröffnet.

Gegenstand der Erfindung ist ein Verfahren zur Isolierung von aromatischen Monoaminen in reiner oder angereicherter Form als Lösung in einem hydrophoben Lösungsmittel oder, in zumindest teilweise protonierter Form, als wäßrige Lösung aus einem, mindestens zwei unterschiedliche aromatische Monoamine enthaltenden

Amingemisch, dadurch gekennzeichnet, daß man in einem ersten Arbeitsgang das Ausgangsgemisch in einem zweiphasigen System, bestehend aus

a) einer hydrophoben Lösungsmittelphase, die gegebenenfalls Monoamin der als wäßrige Lösung in reiner oder angereicherter Form zu gewinnenden Art enthält, und

b) einer wäßrigen Lösung einer starken, bezogen auf die Gesamtmenge der vorliegenden Amine in einer unterstöchiometrischen Menge vorliegenden Säure, die gegebenenfalls Monoamin der als Lösung in hydrophobem Lösungsmittel in reiner oder angereicherter Form zu gewinnenden Art enthält,

unter Durchmischung der Phasen verteilt und die hierbei anfallenden Phasen nach ihrer Trennung isoliert, wonach gegebenenfalls in einem zweiten Arbeitsgang die Lösungsmittelphase erneut mit einer wäßrigen Phase der genannten Art und/oder die wäßrige Phase mit einer Lösungsmittelphase der genannten Art extrahiert wird.

Unter "aromatischen Monoaminen" sind im Rahmen der Erfindung insbesondere Verbindungen der Formel

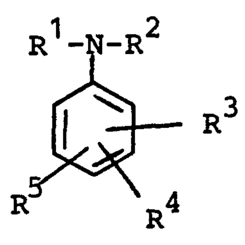

zu verstehen, wobei in dieser Formel

$R^1$ und $R^2$ für gleiche oder verschiedene Reste stehen und Wasserstoff oder Alkylgruppen mit 1 bis 4 Kohlenstoffatomen bedeuten und

$R^3$,$R^4$ und $R^5$ für gleiche oder verschiedene Reste stehen und Wasserstoff, gegebenenfalls über Sauerstoff- oder Schwefelbrücken gebundene Alkylgruppen mit 1 bis 4 Kohlenstoffatomen, Benzyl-, Halogen-, insbesondere Chlor-Substituenten, Trifluormethyl- oder Nitrogruppen bedeuten, mit der Maßgabe, daß höchstens einer der Reste $R^3$ bis $R^5$ für eine Benzyl- oder Nitrogruppe steht.

Das erfindungsgemäße Verfahren ist besonders bevorzugt zur Auftrennung von solchen Amingemischen geeignet, deren Einzelkomponenten der genannten allgemeinen Formel entsprechen, wobei

$R^1$ und $R^2$ für gleiche oder verschiedene Reste stehen und Wasserstoff, Methyl- oder Ethylgruppen bedeuten und

$R^3$,$R^4$ und $R^5$ für gleiche oder verschiedene Reste stehen und Wasserstoff, Methyl- oder Ethylgruppen bzw. Chlorsubstituenten bedeuten, mit der Maßgabe das einer, vorzugsweise zwei der Reste $R^3$ bis $R^5$ für Wasserstoff stehen.

Typische binäre Gemische von aromatischen Monoaminen, die erfindungsgemäß in ihre Bestandteile zerlegt werden können sind in der nachstehenden Tabelle aufgelistet.

**Tabelle**

| Amin 1 | Amin 2 | $pK_B1$ | $pK_B2$ | stärkere Base geht in org. Phase | stärkere Base geht in wäßrige Phase |
|---|---|---|---|---|---|
| Anilin | o-Toluidin | 9,9 | 10,1 | | X |
| Anilin | m-Toluidin | 9,9 | 9,8 | X | |
| Anilin | p-Toluidin | 9,9 | 9,5 | | X |
| Anilin | o-Ethylanilin | 9,9 | 10,2 | | X |
| Anilin | o-Isopropyl-anilin | 9,9 | 10,1 | | X |
| Anilin | N-Methylanilin | 9,9 | 9,9 | | X |
| Anilin | N,N-Dimethyl-anilin | 9,9 | 9,4 | X | |
| Anilin | 2,4-Dimethyl-anilin | 9,9 | 9,6 | X | |
| Anilin | p-Ethylanilin | 9,9 | 9,5 | X | |
| o-Toluidin | m-Toluidin | 10,1 | 9,8 | | X |
| o-Toluidin | p-Toluidin | 10,1 | 9,5 | | X |
| o-Toluidin | 2,6-Methyl-ethylanilin | 10,1 | 10,8 | | X |
| o-Toluidin | o-Ethylanilin | 10,1 | 10,2 | | X |
| m-Toluidin | p-Toluidin | 9,8 | 9,5 | | X |

<u>Tabelle</u> (Fortsetzung)

| Amin 1 | Amin 2 | $pK_B1$ | $pK_B2$ | <u>stärkere</u> Base geht in org. Phase | wäßrige Phase |
|---|---|---|---|---|---|
| o-Ethylanilin | p-Ethylanilin | 10,2 | 9,5 | | X |
| o-Chloranilin | p-Chloranilin | 12,1 | 10,6 | | X |
| o-Methoxy-anilin | p-Methoxy-anilin | 10,0 | 9,7 | | X |
| N-Methyl-anilin | N,N-Dimethyl-anilin | 9,9 | 9,4 | X | |
| N-Ethylanilin | N-Ethyl-2-methyl-anilin | 9,2 | 9,4 | | X |
| N,N-Dimethyl-anilin | N-Ethylanilin | 9,4 | 9,2 | | X |
| 2,6-Methyl-ethylanilin | 2,6-Diethyl-anilin | 10,8 | 10,4 | X | |
| 2,3-Xylidin | 3,4-Xylidin | 9,7 | 9,3 | | X |
| 2,3-Xylidin | 3,5-Xylidin | 9,7 | 9,6 | | X |
| 2,4-Xylidin | 2,5-Xylidin | 9,6 | 9,9 | | X |
| 2,4-Xylidin | 2,6-Xylidin | 9,6 | 9,8 | | X |
| 2,5-Xylidin | 2,6-Xylidin | 9,9 | 9,8 | X | |
| 3,4-Xylidin | 3,5-Xylidin | 9,3 | 9,6 | | X |

Die Frage der Verteilung derartiger binärer Gemische in einem Zweiphasensystem der nachstehend beschriebenen Art ist überraschenderweise von der jeweiligen Basizität der Einzelkomponenten weitgehend unabhängig. Obwohl eigentlich zu erwarten war, daß die jeweils stärkere Base vorzugsweise in der wäßrigen Phase angereichert wird, ist dies, wie aus der Tabelle ersichtlich, nicht immer der Fall. Vor der Durchführung des erfindungsgemäßen Verfahrens wird es somit gegebenenfalls erforderlich sein, in einem kurzen Testversuch festzustellen, ob die in reiner bzw. angereicherter Form zu isolierende Base vorzugsweise in der organischen Phase bzw. in der wäßrigen Phase angereichert, d.h. letztendlich als organische bzw. wäßrige Lösung isoliert wird.

Das erfindungsgemäße Verfahren eignet sich insbesondere zur Auftrennung von Gemischen von aromatischen Monoaminen, die der obengenannten allgemeinen Formel entsprechen. Das erfindungsgemäße Verfahren eignet sich jedoch gleichermaßen zur Auftrennung anderer aromatischer Monoamine wie z.B. von isomeren bzw. homologen, gegebenenfalls Substituenten der obengenannten Art aufweisenden Aminonaphthalinen bzw. Aminoanthracenen.

Eine zur Durchführung des erfindungsgemäßen Verfahrens geeignete Vorrichtung ist beispielhaft in der Zeichnung dargestellt. In dieser Zeichnung bedeuten

(1) einen Behälter für hydrophobes Lösungsmittel;

(2) einen Behälter für aufzutrennendes Amingemisch;

(3) einen Behälter für wäßrige Säure;

(4) einen Behälter für reines bzw. angereichertes, aus der wäßrigen Phase anfallendes Monoamin;

(5) einen Behälter für reines bzw. angereichertes, aus der organischen Phase anfallendes Monoamin;

A eine vorzugsweise mehrstufig arbeitende Extraktionsapparatur für die Durchführung der Verfahrensstufe A;

B eine vorzugsweise mehrstufig arbeitende Extraktionsapparatur für die Durchführung der Verfahrensstufe B und

C eine vorzugsweise mehrstufig arbeitende Extraktionsapparatur für die Durchführung der Verfahrensstufe C.

Das erfindungsgemäße Verfahren wird vorzugsweise in zwei Arbeitsgängen durchgeführt, wobei der erste Arbeitsgang in der Verfahrensstufe A und der zweite Arbeitsgang in der Verfahrensstufe bzw. den Verfahrensstufen B und/oder C besteht. Das Prinzip des erfindungsgemäßen Verfahrens sei zunächst anhand der Zeichnung für den Fall der Trennung eines binären Amingemischs bestehend aus den Einzelkomponenten X und Y mit der organischen Phase als der spezifisch leichteren näher erläutert:

Eine organische Phase AO 1 bestehend aus hydrophobem Lösungsmittel, bzw. einer Lösung der Gesamtmenge oder eines Teils des zu trennenden Amingemischs XY, sowie gegebenenfalls aus im Kreis geführter, aus der Extraktionsapparatur C stammender organischer Lösung wird in den unteren Teil der Extraktionsapparatur A geleitet, während eine wäßrige Phase AW 1, bestehend aus wäßriger Säure oder einer wäßrig-sauren Lösung des zu trennenden Amingemischs XY bzw. eines Teils hiervon, sowie gegebenenfalls einer im Kreislauf geführten, aus der Extraktionsapparatur B stammenden wäßrigen Phase in den oberen Teil der Extraktionsapparatur A geleitet wird. Die beiden Phasen werden in der Extraktionsapparatur A im Gegenstrom, vorzugsweise mehrstufig durchmischt und wieder getrennt, so daß am oberen Teil der Extraktionsapparatur A eine organische Phase AO 2 und am unteren Teil der Extraktionsapparatur A eine wäßrige Phase AW 2 anfallen. Falls nun beispielsweise das Monoamin X in der organischen Phase und das Monoamin Y in der wäßrigen Phase angereichert wird, ist das Molverhältnis X:Y in der Phase AO 2 größer und in der wäßrigen Phase AW 2 kleiner als in den Phasen AO 1 bzw. AW 1. Je nachdem ob eine Reindarstellung bzw. Anreicherung des Monoamins X oder Y oder von beiden Monoaminen angestrebt wird, werden nun die Phasen AO 2 und/oder AW 2 dem zweiten Arbeitsgang zugeführt, d.h. die Phase AO 2 wird in den unteren Teil der Extraktionsapparatur B und/oder die Phase AW 2 wird in den oberen Teil der Extraktionsapparatur C geführt und dort einer weiteren Extraktion unterzogen.

In der Extraktionsstufe B findet eine Gegenstromextraktion der Phase AO 2 mittels einer wäßrigen Phase BW 1 statt, die in den oberen Teil der Extraktionsapparatur B geleitet wird und aus wäßriger Säure oder einer wäßrigen sauren Lösung von Monoamin X besteht. Durch die Verwendung einer derartigen Lösung anstelle einer reinen wäßrigen Säure als Phase BW 1 kann die Effektivität dieser Stufe im Sinne des erfindungsgemäßen Verfahrens beträchtlich gesteigert werden. Die Menge des gegebenenfalls in der wäßrigen sauren Lösung vorliegenden Monoamis X kann innerhalb weiter Grenzen schwanken, wobei es oftmals zweckmäßig ist, die molare Menge des in der Phase BW 1 vorliegenden Monoamins X so zu bemessen, daß sie der in der Phase AO 2 vorliegenden Restmenge des Monoamins Y zumindest entspricht. Bei sehr geringen Restmengen Monoamin Y in der Phase AO 2 kann es zweckmäßig sein, die Menge des in der wäßrigen Phase BW 1 vorliegenden Monoamins X so zu bemessen, daß sie ein vielfaches der Restmenge des Monoamins Y beträgt. Die optimale Konzentration des in der wäßrigen Phase BW 1 gegebenenfalls vorliegenden Monoamins X kann durch einige orientierende Vorversuche ermittelt werden. Im oberen Teil der Extraktionsapparatur B fällt das Monoamin X in reiner bzw. angereicherter Form als organische Lösung an und wird nach destillativer Trennung von dem Lösungsmittel (nicht gezeichnet) in den Behälter (5) geleitet. Die am unteren Teil der Extraktionsapparatur B anfallende wäßrige Phase BW 2 wird als Teil der wäßrigen Phase AW 1 in die Extraktionsapparatur A zurückgeführt.

Die in den oberen Teil der Extraktionsapparatur C geleitete wäßrige Phase AW 2 wird in der Extraktionsapparatur C im Gegenstrom mit der organischen Phase CO 1 einer vorzugsweise mehrstufigen Gegenstromextraktion unterzogen. Die organische Phase CO 1 besteht aus hydrophobem Lösungsmittel (aus (1)) und gegebenenfalls reinem bzw. angereichertem Monoamin Y, welches beim erfindungsgemäßen Verfahren in der wäßrigen Phase in reiner oder angereicherter Form anfällt. Während es durchaus möglich ist, in der Extraktionsstufe B als Extraktionsmittel reine wäßrige Säure zu verwenden, ist es im Falle der Extraktionsstufe C in der Regel angezeigt, d.h. besonders bevorzugt, als Extraktionsmittel anstelle eines reinen hydrophoben Lösungsmittels eine Lösung von reinem bzw. angereichertem Monoamin Y in einem hydrophoben Lösungsmittel einzusetzen. Lediglich in jenen Fällen, in denen die wäßrige Phase AW 2 eine hohe Konzentration an freiem Amin aufweist, ist die Verwendung von reinem hydrophobem Lösungsmittel als Extraktionsmittel für die Stufe C denkbar. Ein hoher Gehalt der wäßrigen Phase AW 2 an freiem Amin kann beispielsweise durch eine Teilneutralisation der die Extraktionsstufe A verlassenden wäßrigen Phase eingestellt werden. Vorzugsweise entspricht die molare Menge des in der organischen Phase CO 1 vorliegenden Amins Y mindestens der molaren Restmenge des in der wäßrigen Phase AW 2 vorliegenden protonierten und gegebenenfalls freien Amins X. Vorzugsweise wird eine 1- bis 10-fach molare

Menge des Amins Y, bezogen auf die Menge des in der Phase AW 2 vorliegenden protonierten und gegebenenfalls freien Amins X zur Herstellung der Phase CO 1 verwendet. Die optimale Menge des Amins Y in der Phase CO 1 kann ebenfalls durch orientierende Vorversuche ermittelt werden. Die Herstellung der vorzugsweise als Phase CO 1 verwendeten Lösung des Amins Y in dem hydrophoben Lösungsmittel kann, wie aus der Zeichnung ersichtlich, durch Vermischen der Einzelkomponenten (aus (1) und (4)) oder auch (nicht gezeichnet) durch eine Nachextraktion der die Stufe C verlassenden wäßrigen Phase, gegebenenfalls nach erfolgter Teilneutralisation, mit reinem Lösungsmittel erhalten werden. Das Ergebnis der Gegenstromextraktion in der Extraktionsapparatur C ist die am unteren Teil anfallende wäßrige Phase CW 2 und die am oberen Teil anfallende organische Phase CO 2. Die wäßrige Phase CW 2 besteht aus reinem bzw. angereichertem Amin Y in zumindest teilweise protonierter Form und wird nach Neutralisation der Säure und Abtrennung der hierbei entstehenden wäßrigen Phase (nicht gezeichnet) in den Lagertank (4) geleitet. Die organische Phase CO 2 wird als Teil der organischen Phase AO 1 in den unteren Teil der Extraktionsapparatur A zurückgeführt.

Falls auf eine Reindarstellung oder weitere Anreicherung des in der organischen Phase AO 2 vorliegenden Amins X oder des in der Phase AW 2 vorliegenden Amins Y verzichtet wird, können diese Phasen unter Ausschaltung der Verfahrensstufen B oder C direkt zwecks Gewinnung der in ihnen enthaltenen Amingemische aufgearbeitet werden.

Die Frage, in wieweit bei der geschilderten Durchführung des erfindungsgemäßen Verfahrens unter Verwendung eines binären Gemischs XY die Amine X und Y in reiner oder nur in angereicherter Form (im Vergleich zum Ausgangsgemisch XY) anfallen, hängt von der Zusammensetzung des Ausgangsgemischs XY und den einzelnen Parametern des Verfahrens wie z.B. dem Wirkungsgrad der eingesetzten Extraktionsapparaturen und den Mengenverhältnissen und Zusammensetzungen der einzelnen Phasen ab. Falls beispielsweise ein Ausgangsgemisch aus 5 Mol X und 95 Mol Y zum Einsatz gelangt, kann es zweckmäßig sein, zunächst ein Gemisch aus X und Y im Molverhältnis 1:1 herzustellen, und dieses Gemisch erneut zur Reindarstellung des Amins X erfindungsgemäß zu behandeln.

Das erfindungsgemäße Verfahren ist selbstverständlich nicht auf die Auftrennung von binären Gemischen XY beschränkt, sondern eignet sich ebenfalls zur Auftrennung von komplexen Gemischen, beispielsweise von Dreiergemischen XYZ (z.B. o-, m- und p-Toluidin), wobei je nach Zusammensetzung des Ausgangsgemischs und den oben beispielhaft genannten Prozeßparametern zunächst beispielsweise reines X als organische Phase anfallen kann, während ein Gemisch aus Y und Z in der wäßrigen Phase gewonnen wird, welches dann seinerseits erneut erfindungsgemäß behandelt werden könnte. Es ist auch denkbar, daß im wesentlichen zunächst ein Gemisch aus X und Y als organische Phase und ein Gemisch aus Y und Z erhalten werden, die dann ihrerseits erneut erfindungsgemäß behandelt werden könnten.

Als hydrophobe Lösungsmittel sind alle mit Wasser nicht mischbaren inerten organischen Lösungsmittel geeignet, in denen sich die Arylamingemische hinreichend lösen. Als besonders vorteilhaft hat es sich erwiesen, Lösungsmittel zu verwenden, die höher sieden als das mit der organischen Phase abgetrennte Arylamin bzw. Arylamingemisch, da in diesem Falle bei der Aufarbeitung das Amin vom Lösungsmittel abdestilliert und dieses gegebenenfalls ohne Destillation wieder eingesetzt werden kann. Geeignete Lösungsmittel sind z.B. aliphatische Kohlenwasserstoffe wie Hexan, Testbenzin, cycloaliphatische Kohlenwasserstoffe wie Cyclopentan, Cyclohexan, Methylcyclohexan, halogenierte Kohlenwasserstoffe wie Methylenchlorid, Chloroform, Tetrachlorkohlenstoff, Dichlorethan, Trichlorethan, Tetrachlorethan, Chlorbenzol, Dichlorbenzol oder aromatische Kohlenwasserstoffe wie Benzol, Toluol, Xylole, höhersiedende aromatische Kohlenwasserstofflösungsmittel oder Nitrobenzol. Zu der Gruppe der hochsiedenden Lösungsmittel, bei denen ein Abdestillieren des mit der organischen Phase abgetrennten Amins möglich ist und die deshalb bevorzugt sind, gehören z.B. Dodecylbenzol, Diphenyl und substituierte Diphenyle, Methylnaphthalin, Chlornaphthalin, Diphenylmethan und substituierte Diphenylmethane, Benzylnaphthalin und substituierte Benzylnaphthaline, und Diphenylether. Außer den reinen Lösungsmitteln können bei dem erfindungsgemäßen Verfahren auch geeignete Lösungsmittelgemische eingesetzt werden. Im Extremfall kann auch das aufzutrennende Amingemisch selbst die Funktion des Lösungsmittels übernehmen, so daß bei

0161600

der Durchführung des erfindungsgemäßen Verfahrens keine Fremdlösungsmittel zum Einsatz gelangen. Dies ist jedoch weniger bevorzugt. Vorzugsweise stellen die einzelnen organischen Phasen 5 bis 60 gew.-%ige Lösungen der Amine bzw. Amingemische in den beispielhaft genannten Lösungsmitteln dar.

Als Säuren werden mittelstarke bis starke Säuren, d.h. solche eines pKa-Werts von unter 3 eingesetzt. Beispiele geeigneter Säuren sind Salzsäure, Schwefelsäure, Phosphorsäure, Trifluoressigsäure oder Benzolsulfonsäure. Im allgemeinen werden 0,01- bis 5-molare, vorzugsweise 0,1- bis 3,5-molare und besonders bevorzugt 0,5- bis 2,5-molare wäßrige Säuren verwendet. Wesentlich ist lediglich, daß die Säure bezüglich ihrer Art und Konzentration so gewählt wird, daß die entsprechenden Aminsalze in der wäßrigen Phase unter den Bedingungen des erfindungsgemäßen Verfahrens löslich sind.

Die Volumenverhältnisse zwischen organischer und wäßriger Phase können bei allen Stufen des erfindungsgemäßen Verfahrens, insbesondere in Abhängigkeit von der Zusammensetzung des aufzutrennenden Amingemischs, innerhalb weiter Grenzen von beispielsweise 1:10 bis 10:1, vorzugsweise 1:5 bis 5:1 schwanken. Die Säuremenge wird vorzugsweise bei allen Stufen des erfindungsgemäßen Verfahrens so gewählt, daß pro Mol als wäßrige Phase abzutrennendes Amin (Phasen AW 2, BW 2 bzw. CW 2) in den entsprechenden wäßrigen Phasen 1 bis 10 Mol Säure vorliegen doch kann für bestimmte Anwendungsfälle auch mit Molverhältnissen außerhalb dieser Grenzen gearbeitet werden. Ein hoher Säureüberschuß innerhalb des genannten Bereichs ist insbesondere dann angezeigt, wenn eine geringe Menge eines Amins über die wäßrige Phase von einer großen Menge eines aus der organischen Phase zu gewinnenden Amins entfernt werden soll.

Die Bauart der beim erfindungsgemäßen Verfahren eingesetzten Extraktionsapparaturen ist von untergeordneter Bedeutung. Es können die in der chemischen Technologie für entsprechende Trennungsoperationen üblichen Vorrichtungen zum Einsatz gelangen. Beispiele hierfür sind mehrstufig arbeitende Gegenstromextraktionskolonnen oder auch in Serie geschaltete Mischer-Scheider-Batterien. Vorzugsweise wird das erfindungsgemäße Verfahren unter Verwendung von 5- bis 15-stufig arbeitenden Gegenstromextraktionskolonnen durchgeführt.

Die Durchführbarkeit des erfindungsgemäßen Verfahrens ist selbstverständlich nicht auf die zwingende Verwendung aller in der Zeichnung lediglich beispielhaft offenbarten Details angewiesen. So ist es beispielsweise nicht zwingend erforderlich, die wäßrige Phase BW 2 der Verfahrensstufe B oder die organische Phase CO 2 der Verfahrensstufe C in die wäßrige Ausgangsphase AW 1 bzw. die organische Ausgangsphase AO 1 zurückzuführen, falls für diese Phasen andere sinnvolle Verwendungsmöglichkeiten bestehen. Außerdem ist es selbstverständlich für die Durchführbarkeit des erfindungsgemäßen Verfahrens unwesentlich, ob die organische Phase bzw. wäßrige Phase, die die einzelnen Amine in reiner oder angereicherter Form enthalten (Phasen BO 2 bzw. CW 2) wie beschrieben durch Entfernung des Lösungsmittels bzw. durch Neutralisation der Säuren und Entfernung des Wassers aufgearbeitet

werden, oder ob diese Phasen als solche einer denkbaren weiteren Verwendung zugeführt werden.

Bei allen Verfahrensstufen wird im allgemeinen unter solchen Temperatur- und Druckbedingungen gearbeitet, innerhalb derer beide Phasen flüssig sind. Vorzugsweise wird das Verfahren innerhalb des Temperaturbereichs von 15°C bis unterhalb 100°C, vorzugsweise von 25 bis 95°C unter Normaldruck durchgeführt. Es kann zweckmäßig sein, die einzelnen Verfahrensschritte bei unterschiedlichen Temperaturen durchzuführen. Die Anwendung von Über- oder Unterdruck ist selbstverständlich nicht prinzipiell ausgeschlossen.

Die vorzugsweise durchzuführende Aufarbeitung der organischen Phasen AO 2 bzw. BO 2 erfolgt im allgemeinen durch Entfernung geringer Säuremengen durch Neutralisation mit Basen (z.B. Natronlauge), über Ionenaustauscher oder durch Waschen mit Wasser und anschließende destillative Trennung in Lösungsmittel und Verfahrensprodukt.

Die Aufarbeitung der wäßrigen Phasen AW 2 bzw. CW 2 erfolgt im allgemeinen durch Neutralisation der Säure mit starken Basen wie Natriumhydroxid oder Kaliumhydroxid, Abtrennung des dabei freigesetzten Monoamins und gegebenenfalls dessen destillative Reinigung.

Im übrigen kann es sich oftmals als zweckmäßig herausstellen, einen Teil der in den wäßrigen Phasen BW 2 oder AW 2 (bei Durchführung des Verfahrensschrittes C) enthaltenen Säure vor deren Rückführung in die Phase AW 1 bzw. Weiterleitung in die Extraktionsstufe C an geeigneter Stelle in ihre neutralen Salze durch

Zugabe einer entsprechenden Menge einer starken Base wie Natrium- oder Kaliumhydroxid zu überführen, um so die Konzentration an Säure entsprechend den obengemachten Ausführungen einzustellen. Daraus ergibt sich zwangsläufig, daß es zweckmäßig sein kann, die einzelnen Verfahrensschritte bei voneinander abweichenden Säure-Molaritäten der wäßrigen Phasen durchzuführen, was außer durch Teilneutralisation auch durch Hinzufügen oder Abdestillieren von Wasser oder Hinzufügen von Säure zwischen den Verfahrensschritten erreicht werden kann.

Mit dem erfindungsgemäßen Verfahren ist es möglich, gegebene Gemische von aromatischen Monoaminen in Gemische anderer Zusammensetzung der Einzelkomponenten aufzutrennen, aus gegebenen Gemischen von aromatischen Monoaminen einzelne der Komponenten in reiner Form abzutrennen, bzw. derartige Gemische vollständig in die reinen Einzelkomponenten zu zerlegen bzw. aus Gemischen von aromatischen Monoaminen, in denen eine Komponente in angereicherter Form vorliegt (technische Reinheit), diese Komponente in beliebigem Reinheitsgrad (hochrein bzw. chemisch rein) zu isolieren. Das erfindungsgemäße Verfahren eignet sich insbesondere auch zur Auftrennung von solchen Amingemischen, die auf anderem Wege nicht in die Einzelkomponenten zerlegt werden können.

Beispiel 1 (vgl. Zeichnung)

Dieses Beispiel zeigt die Trennung eines Gemischs aus gleichen Teilen ortho- und para-Toluidin:

A. In einer Verfahrensstufe A bestehend aus einer mehrstufig wirkenden Siebboden-Extraktionskolonne einer Höhe von 400 cm und einer lichten Weite von 5 cm werden bei 85-95°C eine organische Phase AO 1 und eine wäßrige Phase AW 1 kontinuierlich im Gegenstrom aneinander vorbeigeführt.

Die organische Phase AO 1 hat folgende Zusammensetzung:

| | | |
|---|---|---|
| AO 1: | 225 g | ortho-Toluidin |
| | 225 g | para-Toluidin |
| | 1700 g | ortho-Xylol |

Dies entspricht einer Menge von 2150 g pro Stunde der genannten organischen Lösung AO 1.

Die wäßrige Phase AW 1 hat folgende Zusammensetzung:

| | | |
|---|---|---|
| AW 1: | 321 g | ortho-Toluidin |
| | 321 g | para-Toluidin |
| | 188 g | Chlorwasserstoff |
| | 1920 g | Wasser |

0161600

Dies entspricht einer Menge von 2750 g dieser wäßrigen Lösung pro Stunde.

Die organische Phase AO 1 wird kontinuierlich in den unteren Teil und die wäßrige Phase AW 1 kontinuierlich in den oberen Teil der Extraktionskolonne eingespeist.

Im Gleichgewichtszustand hat die aus dem oberen Teil der Kolonne kontinuierlich ablaufende organische Phase AO 2 folgende durchschnittliche Zusammensetzung:

| | | |
|---|---|---|
| AO 2: | 376 | g ortho-Toluidin |
| | 96 | g para-Toluidin |
| | 1700 | g ortho-Xylol |

Dies entspricht einer Menge von 2172 g der genannten organischen Lösung AO 2 pro Stunde.

Die am unteren Ende der Kolonne kontinuierlich ablaufende wäßrige Phase AW 2 hat folgende Zusammensetzung:

| | | |
|---|---|---|
| AW 2: | 170 | g ortho-Toluidin |
| | 450 | g para-Toluidin |
| | 188 | g Chlorwasserstoff |
| | 1920 | g Wasser |

Dies entspricht einer Menge von 2728 g der wäßrigen Phase AW 2 pro Stunde.

B. Die organische Phase AO 2 aus der Stufe A wird in einer zweiten Verfahrensstufe B bestehend aus einer mehrstufig wirkenden Siebboden-Extraktionskolonne der Höhe 400 cm und der lichten Weite 5 cm bei 85 bis 95°C mit einer wäßrigen Phase BW 1 im Gegenstrom extrahiert, wobei die organische Phase AO 2 in den unteren und die wäßrige Phase BW 1 in den oberen Teil der Extraktionskolonne kontinuierlich eingespeist wird. Die wäßrige Phase BW 1 hat folgende Zusammensetzung:

| | |
|---|---|
| BW 1: | 170 g ortho-Toluidin |
| | 50 g Chlorwasserstoff |
| | 511 g Wasser |

Dies entspricht einer Menge von 731 g der wäßrigen Lösung BW 1 pro Stunde.

Die kontinuierlich am oberen Teil der Extraktionskolonne B ablaufende organische Phase BO 2 hat folgende durchschnittliche Zusammensetzung:

| | |
|---|---|
| BO 2: | 450 g ortho-Toluidin |
| | 1700 g ortho-Xylol |

Dies entspricht einer Menge von 2150 g der organischen Lösung BO 2 pro Stunde.

Die organische Phase BO 2 wird anschließend mit Wasser gewaschen und destillativ in ortho-Toluidin und ortho-Xylol aufgetrennt (nicht gezeichnet).

Das Xylol wird in den Lagertank (1) für hydrophobes Lösungsmittel zurückgeleitet, während 170 g/h des ortho-Toluidins zur Bereitung der wäßrigen Phase BW 1 verwendet werden und die Restmenge von 280 g/h eines der Verfahrensprodukte darstellt.

Die am unteren Teil der Extraktionskolonne B ablaufende wäßrige Phase BW 2 hat folgende durchschnittliche Zusammensetzung:

| | | |
|---|---|---|
| BW 2: | 96 g | ortho-Toluidin |
| | 96 g | para-Toluidin |
| | 50 g | Chlorwasserstoff |
| | 511 g | Wasser |

Dies entspricht einer Menge von 753 g der wäßrigen Lösung BW 2 pro Stunde.

Die wäßrige Phase BW 2 wird kontinuierlich durch Abmischen mit frischer wäßriger Salzsäure (aus 3) und frischem Ausgangsgemisch (aus 2) vermischt, so daß die kontinuierlich in die Extraktionskolonne A einzuspeisende wäßrige Phase AW 1 resultiert. Hierbei gelangen ca. 450 g/h des Ausgangsgemischs (ortho- und para-Toluidin im Gewichtsverhältnis 1:1), 138 g/h Chlorwasserstoff und 1409 g/h Wasser zum Einsatz.

C. Die am unteren Teil der Kolonne A kontinuierlich ablaufende wäßrige Phase AW 2 wird in einer 3. Verfahrensstufe C, bestehend aus einer mehrstufig wirkenden Siebboden-Extraktionskolonne entsprechend der Extraktionskolonne B bei 85-95°C mit einer organischen Phase CO 1 im Gegenstrom extrahiert. Die Phase CO 1 hat folgende Zusammensetzung:

CO 1: 350 g para-Toluidin
1150 g ortho-Xylol

Dies entspricht einer Menge von 1500 g der organischen Lösung CO 1 pro Stunde.

Die am unteren Teil der Extraktionskolonne C ablaufende wäßrige Phase CW 2 hat folgende durchschnittliche Zusammensetzung:

CW 2: 630 g para-Toluidin
188 g Chlorwasserstoff
1920 g Wasser

Dies entspricht einer Menge von 2738 g der wäßrigen Lösung CW 2 pro Stunde.

Die wäßrige Lösung CW 2 wird neutralisiert und das nach Phasentrennung erhaltene para-Toluidin durch Destillation gereinigt. Eine Teilmenge (350 g) wird zur Bereitung der organischen Phase CO 1 recyclisiert, die restlichen 280 g stellen das zweite Verfahrensprodukt dar.

Die am oberen Teil der Extraktionskolonne C kontinuierlich ablaufende organische Phase CO 2 hat folgende durchschnittliche Zusammensetzung:

CO 2: 170 g ortho-Toluidin
170 g para-Toluidin
1150 g ortho-Xylol

Dies entspricht einer Menge von 1490 g der organischen Lösung CO 2 pro Stunde.

Die organische Lösung CO 2 wird mit frischem Lösungsmittel (aus 1) und frischem Ausgangsgemisch (aus 2) zur organischen Phase AO 1 vereinigt. Hierzu gelangen 110 g/h des Ausgangsgemischs und 550 g ortho-Xylol zum Einsatz.

Nach dem geschilderten Verfahren werden somit 560 g des Ausgangsgemischs, bestehend aus gleichen Gewichtsteilen ortho- und para-Toluidin, in reines ortho- und para-Toluidin aufgetrennt, wobei die resultierenden Einzelkomponenten bereits vor ihrer destillativen Reindarstellung einen Reinheitsgrad von über 99 % aufweisen.

Beispiel 2

Trennung von ortho- und para-Chloranilin

Die Trennung eines Gemisches aus gleichen Teilen ortho- und para-Chloranilin erfolgt in einer Extraktionsanlage analog Beispiel 1, die Zusammensetzungen und die Mengenströme der jeweiligen Phasen sind im folgenden angegeben.

| | | | | |
|---|---|---|---|---|
| A. | AO 1 | : | 318 g | ortho-Chloranilin |
| | | | 318 g | para-Chloranilin |
| | | | 1912 g | ortho-Xylol |

Dies entspricht einer Menge von 2548 g/h der genannten organischen Lösung AO 1.

| | | | |
|---|---|---|---|
| AW 1 | : | 319 g | ortho-Chloranilin |
| | | 319 g | para-Chloranilin |
| | | 183 g | Chlorwasserstoff |
| | | 1875 g | Wasser |

Dies entspricht einer Menge von 2696 g dieser wässrigen Lösung pro Stunde.

Im Gleichgewichtszustand fallen an :

| | | |
|---|---|---|
| AO 2 : | 587 g | ortho-Chloranilin |
| | 37 g | para-Chloranilin |
| | 1912 g | ortho-Xylol |

Dies entspricht einer Menge von 2536 g der genannten organischen Lösung AO 2 pro Stunde.

| | | |
|---|---|---|
| AW 2 : | 50 g | ortho-Chloranilin |
| | 600 g | para-Chloranilin |
| | 183 g | Chlorwasserstoff |
| | 1875 g | Wasser |

Dies entspricht einer Menge von 2708 g der wäßrigen Phase AW 2 pro Stunde.

B.

| | | |
|---|---|---|
| BW 1 : | 74 g | ortho-Chloranilin |
| | 22 g | Chlorwasserstoff |
| | 221 g | Wasser |

Dies entspricht einer Menge von 317 g der wäßrigen Lösung BW 1 pro Stunde.

| | | |
|---|---|---|
| BO 2 : | 624 g | ortho-Chloranilin |
| | 1912 g | ortho-Xylol |

Dies entspricht einer Menge von 2536 g der organischen Lösung BO 2 pro Stunde. Nach Waschen mit Wasser und Abdestillieren des ortho-Xylols (nicht eingezeichnet) werden 74 g/h des ortho-Chloranilins zur Bereitung der wäßrigen Phase verwendet; die Restmenge von 550 g/h stellt eines der Verfahrensprodukte dar.

| | | |
|---|---|---|
| BW 2 : | 37 g | ortho-Chloranilin |
| | 37 g | para-Chloranilin |
| | 22 g | Chlorwasserstoff |
| | 221 g | Wasser |

Dies entspricht einer Menge von 317 g der wäßrigen Lösung BW 2 pro Stunde.

Die wäßrige Phase BW 2 wird kontinuierlich mit frischer Salzsäure (aus 3) und frischen Ausgangsgemisch (aus 2) versetzt, so daß die kontinuierlich in die Extraktionskolonne A einzuspeisende wäßrige Phase AW 1 resultiert. Hierbei gelangen ca. 564 g/h des Ausgangsgemischs (ortho- und para-Chloranilin im Gewichtsverhältnis 1 : 1 ), 161 g/h Chlorwasserstoff und 1654 g/h Wasser zum Einsatz.

C. CO 1 : 100 g para-Chloranilin
300 g ortho-Xylol

Dies entspricht einer Menge von 400 g der organischen Lösung CO 1 pro Stunde.

CW 2 : 650 g para-Chloranilin
183 g Chlorwasserstoff
1875 g Wasser

Dies entspricht einer Menge von 2708 g der wäßrigen Lösung CW 2 pro Stunde.
Die wäßrige Lösung CW 2 wird neutralisiert; das erhaltene para-Chloranilin kann durch Abtrennen (heiß) oder Absaugen (kalt) isoliert werden. Eine Teilmenge ( 100 g/h ) wird für die Herstellung der organischen CO 1 recyclisiert, die restlichen 550 g stellen das zweite Verfahrensprodukt dar.

CO 2 : 50 g ortho-Chloranilin
50 g para-Chloranilin
300 g ortho-Xylol

Dies entspricht einer Menge von 400 g der organischen Lösung CO 2 pro Stunde.

Die organische Lösung CO 2 wird mit frischem Lösungsmittel (aus 1) und frischem Ausgangsgemisch (aus 2) zur organischen Phase AO 1 vereinigt. Hierzu gelangen 536 g/h des Ausgangsgemisches und 1612 g ortho-Xylol zum Einsatz.

Nach dem geschilderten Verfahren werden somit 1100 g/h des Ausgangsgemisches, bestehend aus gleichen Gewichtsteilen ortho- und para-Chloranilin, in reines ortho- und para-Chloranilin aufgetrennt, wobei die resultierenden Einzelkomponenten einen Reinheitsgrad von über 99 % aufwiesen.

Beispiel 3

Trennung von ortho- und para-Chloranilin

A. In einer Verfahrensstufe A bestehend aus einer einstufigen Mischer-Scheider-Anlage (Normag) werden bei 90 - 95°C kontinuierlich die organische Phase AO 1 und die wäßrige Phase AW 1 gemischt.

Die organische Phase AO 1 hat folgende Zusammensetzung :

| | | |
|---|---|---|
| AO 1 : | 106 g | ortho-Chloranilin |
| | 106 g | para-Chloranilin |
| | 638 g | ortho-Xylol |

Dies entspricht einer Menge von 850 g/h der genannten organischen Lösung AO 1.

Die wäßrige Phase AW 1 hat folgende Zusammensetzung:

| | | |
|---|---|---|
| AW 1 : | 109 g | ortho-Chloranilin |
| | 109 g | para-Chloranilin |
| | 60 g | Chlorwasserstoff |
| | 622 g | Wasser |

Dies entspricht einer Menge von 900 g dieser wäßrigen Lösung pro Stunde.

Die im Gleichgewichtszustand befindlichen Phasen haben nach der Trennung folgende durchschnittliche Zusammensetzung :

| | | |
|---|---|---|
| AO 2 : | 173 g | ortho-Chloranilin |
| | 39 g | para-Chloranilin |
| | 638 g | ortho-Xylol |

Dies entspricht einer Menge von 850 g der genannten organischen Lösung AO 2 pro Stunde.

| | | |
|---|---|---|
| AW 2 : | 42 g | ortho-Chloranilin |
| | 176 g | para-Chloranilin |
| | 60 g | Chlorwasserstoff |
| | 622 g | Wasser |

Dies entspricht einer Menge von 900 g der wäßrigen Phase AW 2 pro Stunde.

B. Die organische AO 2 aus der Stufe A wird kontinuierlich in einer dreistufigen Mischer-Scheider-Anlage bei 90 - 95°C im Gegenstrom mit einer wäßrigen Phase BW 1 folgender Zusammensetzung zusammengebracht :

| | | |
|---|---|---|
| BW 1 : | 21.5 g | Chlorwasserstoff |
| | 222.5 g | Wasser |

Dies entspricht einer Menge von 244 g der wäßrigen Lösung BW 1 pro Stunde.

Die ablaufende Phase BO 2 hat folgende durchschnittliche Zusammensetzung :

| | | |
|---|---|---|
| BO 2 : | 134 g | ortho-Chloranilin |
| | 638 g | ortho-Xylol |

Die entspricht einer Menge von 772 g der organischen Phase BO 2 pro Stunde.

Die organische Phase BO 2 wird mit Wasser gewaschen, zur Aufarbeitung wird ortho-Xylol abdestilliert.

Die ablaufende wässrige Phase BW 2 hat folgende durchschnittliche Zusammensetzung :

| | | |
|---|---|---|
| BW 2 : | 39 g | ortho-Chloranilin |
| | 39 g | para-Chloranilin |
| | 21,5g | Chlorwasserstoff |
| | 222,5g | Wasser |

Dies entspricht einer Menge von 322 g der wäßrigen Lösung BW 2 pro Stunde.

Die wäßrige Phase BW 2 wird kontinuierlich mit frischer Salzsäure und frischem Ausgangsgemisch versetzt, so daß die kontinuierlich in die Mischer-Scheider-Stufe A einzuspeisende wäßrige Lösung AW 1 resultiert. Hierbei gelangen ca. 140 g/h des Ausgangsgemischs (ortho- und para-Chloranilin im Gewichtsverhältnis 1:1), 38,5 g Chlorwasserstoff und 399.5 g Wasser zum Einsatz.

C. Im Verfahrensschritt C - bestehend aus einer dreistufigen Mischer-Scheider-Anlage - wird bei 90 - 95°C die aus dem Verfahrensschritt A stammende wäßrige Phase AW 2 im Gegenstrom mit einer organischen Phase CO 1 extrahiert. Die Phase CO 1 hat folgende Zusammensetzung :

| | | |
|---|---|---|
| CO 1 : | 84 g | para-Chloranilin |
| | 253 g | ortho-Xylol |

Das entspricht einer Menge von 337 g der organischen Lösung CO 1 pro Stunde.

Die ablaufende wäßrige Phase CW 2 hat folgende durchschnittliche Zusammensetzung :

| | | |
|---|---|---|
| CW 2 : | 218 g | para-Chloranilin |
| | 60 g | Chlorwasserstoff |
| | 622 g | Wasser |

Dies entspricht einer Menge von 900 g der wäßrigen Phase CW 2 pro Stunde.

Nach Neutralisation können 218 g para-Chloranilin isoliert werden, wovon 84 g zur Herstellung von CO 1 abgezweigt werden.

Die ablaufende organische Phase CO 2 enthält :

| | | |
|---|---|---|
| CO 2 : | 42 g | ortho-Chloranilin |
| | 42 g | para-Chloranilin |
| | 253 g | ortho-Xylol |

Dies entspricht einer Menge von 337 g der organischen Lösung CO 2 pro Stunde.

Die organische Lösung CO 2 wird mit frischem Lösungsmittel und frischem Ausgangsgemisch zur organischen Phase AO 1 vereinigt.
Hierzu gelangen 128 g/h des Ausgangsgemischs und 550 g/h ortho-Xylol zum Einsatz.

0161600

Beispiel 4

Trennung von N-Methylanilin (Kp 196°C) und N,N-Dimethylanilin (Kp 194°C)

Die Trennung dieses Amingemisches erfolgt in einer Extraktionsanlage analog Beispiel 1, die Zusammensetzung und die Mengenströme der jeweiligen Phasen sind im folgenden angegeben. Als hydrophobes Lösungsmittel wird technisches Methyldiphenylmethan - nachstehend als Lösungsmittel bezeichnet - verwendet.

| | | | | |
|---|---|---|---|---|
| A. | AO 1 : | 272,5 | g | N-Methylanilin |
| | | 296,5 | g | N,N-Dimethylanilin |
| | | 1710 | g | Lösungsmittel |

Dies entspricht einer Menge von 2279 g/h der genannten organischen Lösung AO 1.

| | | | |
|---|---|---|---|
| AW 1 : | 262,5 | g | N-Methylanilin |
| | 308,5 | g | N,N-Dimethylanilin |
| | 182,5 | g | Chlorwasserstoff |
| | 1825,5 | g | Wasser |

Dies entspricht einer Menge von 2579 g dieser wäßrigen Lösung pro Stunde.

0161600

Im Gleichgewichtszustand fallen an :

| | | | |
|---|---|---|---|
| AO 2 : | 153 g | N-Methylanilin |
| | 432 g | N,N-Dimethylanilin |
| | 1710 g | Lösungsmittel |

Dies entspricht einer Menge von 2295 g der gesamten organischen Lösung AO 2 pro Stunde.

| | | |
|---|---|---|
| AW 2 : | 382 g | N-Methylanilin |
| | 173 g | N,N-Dimethylanilin |
| | 182,5g | Chlorwasserstoff |
| | 1825,5g | Wasser |

Dies entspricht einer Menge von 2563 g der wäßrigen Lösung AW 2 pro Stunde.

| | | | |
|---|---|---|---|
| B. | BW 1 : | 346 g | N,N-Dimethylanilin |
| | | 104,5g | Chlorwasserstoff |
| | | 1044 g | Wasser |

Dies entspricht einer Menge von 1494,5 g der wäßrigen Lösung BW 1 pro Stunde.

| | | |
|---|---|---|
| BO 2 : | 5 g | N-Methylanilin |
| | 599 g | N,N-Dimethylanilin |
| | 1710 g | Lösungsmittel |

Dies entspricht einer Menge von 2314 g der organischen Lösung BO 2 pro Stunde.

Aus BO 2 kann N,N-Dimethylanilin mit einer Reinheit von 99 % abdestilliert werden. 346 g/h werden zur Herstellung der wäßrigen Phase BW 1 verwendet, die Restmenge stellt eines der Verfahrensprodukte dar. Das verbleibende Lösungsmittel wird in den Lagertank zurückgeleitet.

| | | | |
|---|---|---|---|
| BW 2 : | 148 | g | N-Methylanilin |
| | 179 | g | N,N-Dimethylanilin |
| | 104,5 | g | Chlorwasserstoff |
| | 1044 | g | Wasser |

Dies entspricht einer Menge von 1475,5 g der wäßrigen Lösung BW 2 pro Stunde.

Die wäßrige Phase BW 2 wird kontinuierlich mit frischer Salzsäure und frischem Ausgangsgemisch (N-Methylanilin und N,N-Dimethylanilin im Molverhältnis 1:1) versetzt, so daß die kontinuierlich in die Extraktionskolonne A einzuspeisende wäßrige Phase AW 1 resultiert. Hierbei gelangen pro Stunde ca. 244 g des obigen Ausgangsgemisches, 78 g Chlorwasserstoff und 781,5 g Wasser zum Einsatz.

C.

| | | | |
|---|---|---|---|
| CO 1 : | 306 | g | N-Methylanilin |
| | 918 | g | Lösungsmittel |

Dies entspricht einer Menge von 1224 g der organischen Lösung CO 1 pro Stunde.

| | | | |
|---|---|---|---|
| CW 2 : | 530 | g | N-Methylanilin |
| | 6 | g | N,N-Dimethylanilin |
| | 182,5 | g | Chlorwasserstoff |
| | 1825,5 | g | Wasser |

Dies entspricht einer Menge von 2544 g der wäßrigen Lösung CW 2 pro Stunde.
Die wäßrige Lösung CW 2 wird neutralisiert; N-Methylanilin kann durch Phasentrennung mit einer Reinheit von 99 % isoliert werden. Eine Teilmenge (306 g/h) wird zur Bereitung der organischen Phase CO 1 recyclisiert, die Restmenge stellt das zweite Verfahrensprodukt dar.

| | | | |
|---|---|---|---|
| CO 2 : | 158 | g | N-Methylanilin |
| | 167 | g | N,N-Dimethylanilin |
| | 918 | g | Lösungsmittel |

Dies entspricht einer Menge von 1243 g der organischen Lösung CO 2 pro Stunde.
Die organische Lösung CO 2 wird mit frischem Lösungsmittel und frischem Ausgangsgemisch (N-Methylanilin und N,N-Dimethylanilin im Molverhältnis 1:1) zur organischen Phase AO 1 vereinigt. Hierzu gelangen pro Stunde ca. 244g Ausgangsgemisch und 792 g Lösungsmittel zum Einsatz.

0161600

Beispiel 5

Trennung eines Amingemisches mit Mono- und Diisopropylanilin als Hauptkomponenten

A. Die Verfahrensstufen A und B bestehen aus einer insgesamt 10-stufigen Mischer-Scheider-Anlage (Normag), deren organische Phasen hintereinandergeschaltet sind. Im dreistufigen Teil A wird bei 90 - 95°C die organische Phase AO 1 im Gegenstrom mit einer wäßrigen Phase AW 1 zusammengebracht.

AO 1 : 1840 g/h Amingemisch, welches sich aus dem Konzentrat der Verfahrensstufe C (68,5 g/h) und 1771,5 g/h des zu trennenden Mono-/Diisopropylanilingemisches ( Zusammensetzung nach Gaschromatographie (Flächen-%) : 0,4% Anilin, 3,7% nicht ident. Isomere, 14,8% 2-Isopropylanilin, 72,6% 2,6-Diisopropylanilin, 4,5% N-Propylanilin, 4,0% Triisopropylanilin) zusammensetzt. 2,6-Diisopropylanilin und N-Propylanilin wurden gaschromatographisch nicht vollständig aufgetrennt, sie werden deshalb im folgenden als Summe angegeben. Ihre relativen Anteile werden durch das Trennverfahren nicht wesentlich verändert.

AW 1 : Die wäßrige Phase AW 1 besteht aus dem Ablauf der Stufe B, welchem pro Stunde 1195,5 g Wasser und 54,5 g HCl zugesetzt werden.

Die im Gleichgewicht ablaufende wäßrige Phase AW 2 hat folgende durchschnittliche Zusammensetzung :

AW 2 : 355 g Amingemisch mit
2.4 % Anilin
6.4 % nicht ident. Isomere
76.8 % 2-Isopropylanilin
14.4 % 2,6-Diisopropylanilin und N-Propylanilin
0.1 % Triisopropylanilin
107,5g Chlorwasserstoff
2860,5g Wasser

Dies entspricht einer Menge von 3323 g der wäßrigen Phase AW 2 pro Stunde.

Die im Gleichgewicht ablaufende organische Phase AO 2 gelangt direkt in die Verfahrensstufe B.

B. Die Verfahrensstufe B besteht aus einer 7-stufigen Mischer-Scheider-Anlage, die bei 90-95°C betrieben wird. In Stufe 1 wird die organische Phase AO 2 eingespeist; diese wird im Gegenstrom mit der wäßrigen Phase BW 1 zusammengebracht, die sich aus BW 1a und BW 1b zusammensetzt. BW 1a (5oo g/h Wasser) wird in Stufe 7 eingespeist, BW 1b (54,5 g/h Chlorwasserstoff, 1195,5 g/h Wasser) wird zwischen der 5. und 6. Stufe der ablaufenden Phase BW 1a zugegeben.

Die ablaufende organische Phase BO 2 hat folgende durchschnittliche Zusammensetzung :

| | | | |
|---|---|---|---|
| BO 2 : | 1485 g | Amingemisch mit | |
| | | 3,0 % | nicht ident. Isomere |
| | | 92,1 % | 2,6-Diisopropylanilin und N-Propylanilin |
| | | 4,9 % | Triisopropylanilin |
| | 1,5g | Chlorwasserstoff | |
| | 30,5g | Wasser | |

Dies entspricht einer Menge von 1517 g der organischen Phase BO 2 pro Stunde.

BO 2 wird mit wäßriger Natronlauge neutralisiert und stellt - nach Abtrennen der wäßrigen Phase - eines der Verfahrensprodukte dar.

Der im Gleichgewicht befindliche Mengenstrom BW 2 (Auslauf 1. Stufe der Verfahrensstufe B) wird wie beschrieben mit verd. Salzsäure zum Mengenstrom AW 1 ergänzt.

C. Im Verfahrensschritt C - bestehend aus einer fünfstufigen Mischer-Scheider-Anlage - wird bei 90-95°C die aus dem Verfahrensschritt A stammende wäßrige Phase AW 2 im Gegenstrom mit einer organischen Phase CO 1 extrahiert.
Die Phase CO 1 hat folgende Zusammensetzung :

| | | |
|---|---|---|
| CO 1 : | 68,5 g | 2-Isopropylanilin |
| | 680,0 g | ortho-Xylol |

Dies entspricht einer Menge von 748,5 g der organischen Lösung CO 1 pro Stunde.

Im Gleichgewichtszustand fallen an :

| | | | |
|---|---|---|---|
| CW 2 | : | 322,5 g | Amingemisch mit |
| | | | 2,6% Anilin |
| | | | 6,2% nicht ident. Isomere |
| | | | 90,7% 2-Isopropylanilin |
| | | | 0,5% Triisopropylanilin |
| | | 107,5 g | Chlorwasserstoff |
| | | 2860,5 g | Wasser |

Dies entspricht einer Menge von 3290,5 g der wäßrigen Phase CW 2 pro Stunde. Von CW 2 wird das Amin nach Neutralisieren abgetrennt, eine Teilmenge (68,5 g/h) kann zur Herstellung der organischen Phase CO 1 recycisiert werden, die restlichen 254 g stellen das zweite Verfahrensprodukt dar.

| | | | |
|---|---|---|---|
| CO 2 | : | 68,5 g | Amingemisch mit |
| | | | 4,0% nicht ident. Isomere |
| | | | 23,6% 2-Isopropylanilin |
| | | | 72,4% 2,6-Diisopropylanilin und N-Propylanilin |
| | | 680 g | ortho-Xylol |

Dies entspricht einer Menge von 748,5 g der organischen Lösung CO 2 pro Stunde. Das nach Abdestillieren des Lösungsmittels verbleibende Amin wird mit Ausgangsgemisch zum Mengenstrom AO 1 ergänzt.

Geringe Abweichungen in den Mengenbilanzen sind durch Ungenauigkeiten der gaschromatographischen und titrimetrischen Analysen bedingt.

## Patentansprüche

1. Verfahren zur Isolierung von aromatischen Monoaminen in reiner oder angereicherter Form als Lösung in einem hydrophoben Lösungsmittel oder, in zumindest teilweise protonierter Form, als wäßrige Lösung aus einem, mindestens zwei unterschiedliche aromatische Monoamine enthaltenden Amingemisch, dadurch gekennzeichnet, daß man in einem ersten Arbeitsgang das Ausgangsgemisch in einem zweiphasigen System, bestehend aus

   a) einer hydrophoben Lösungsmittelphase, das gegebenenfalls Monoamin der als wäßrige Lösung in reiner oder angereicherter Form zu gewinnenden Art enthält, und

   b) einer wäßrigen Lösung einer starken, bezogen auf die Gesamtmenge der vorliegenden Amine in einer unterstöchiometrischen Menge vorliegenden Säure, die gegebenenfalls Monoamin der als Lösung in hydrophobem Lösungsmittel in reiner oder angereicherter Form zu gewinnenden Art enthält,

   unter Durchmischung der Phasen verteilt und die hierbei anfallenden Phasen nach ihrer Trennung isoliert, wonach gegebenenfalls in einem zweiten Arbeitsgang die Lösungsmittelphase erneut mit einer wäßrigen Phase der genannten Art und/oder die wäßrige Phase mit einer Lösungsmittelphase der genannten Art extrahiert wird.

2. Verfahren gemäß Anspruch 1, dadurch gekennzeichnet, daß man die Durchmischung und anschließende Trennung der Phasen des ersten Arbeitsgangs in einem ein- oder mehrstufigen Gegenstromextraktor durchführt.

3. Verfahren gemäß Anspruch 1 und 2, dadurch gekennzeichnet, daß man die beim ersten Arbeitsgang erhaltene Lösungsmittelphase einer mehrstufigen Extraktion mit einer wäßrigen Phase der in Anspruch 1 genannten Art nach dem Gegenstromprinzip unterwirft.

4. Verfahren gemäß Anspruch 1 und 2, dadurch gekennzeichnet, daß man die beim ersten Arbeitsgang erhaltene wäßrige Phase mit einer Lösungsmittelphase, der Monoamin der als wäßrige Lösung in reiner oder angereicherter Form zu gewinnenden Art in einer molaren Menge zugesetzt worden ist, die der Menge des aus der wäßrigen Phase zu entfernenden Monoamins zumindest entspricht, einer mehrstufigen Extraktion nach dem Gegenstromprinzip unterwirft.

5. Verfahren gemäß Anspruch 1 und 2, dadurch gekennzeichnet, daß man vor der Durchmischung der Phasen das Ausgangsgemisch in der Lösungsmittelphase oder der sauren wäßrigen Phase löst oder in zwei Teilmengen unterteilt und die Teilmengen in jeweils einer der beiden Phasen löst.

6. Verfahren zur Isolierung von aromatischen Monoaminen in reiner oder angereicherter Form, dadurch gekennzeichnet, daß man die gemäß Ansprüchen 1 bis 5 erhaltenen Lösungen, gegebenenfalls nach Neutralisation der vorliegenden Säure in an sich bekannter Weise destillativ und/oder durch Abtrennung der wäßrigen Phase aufarbeitet.

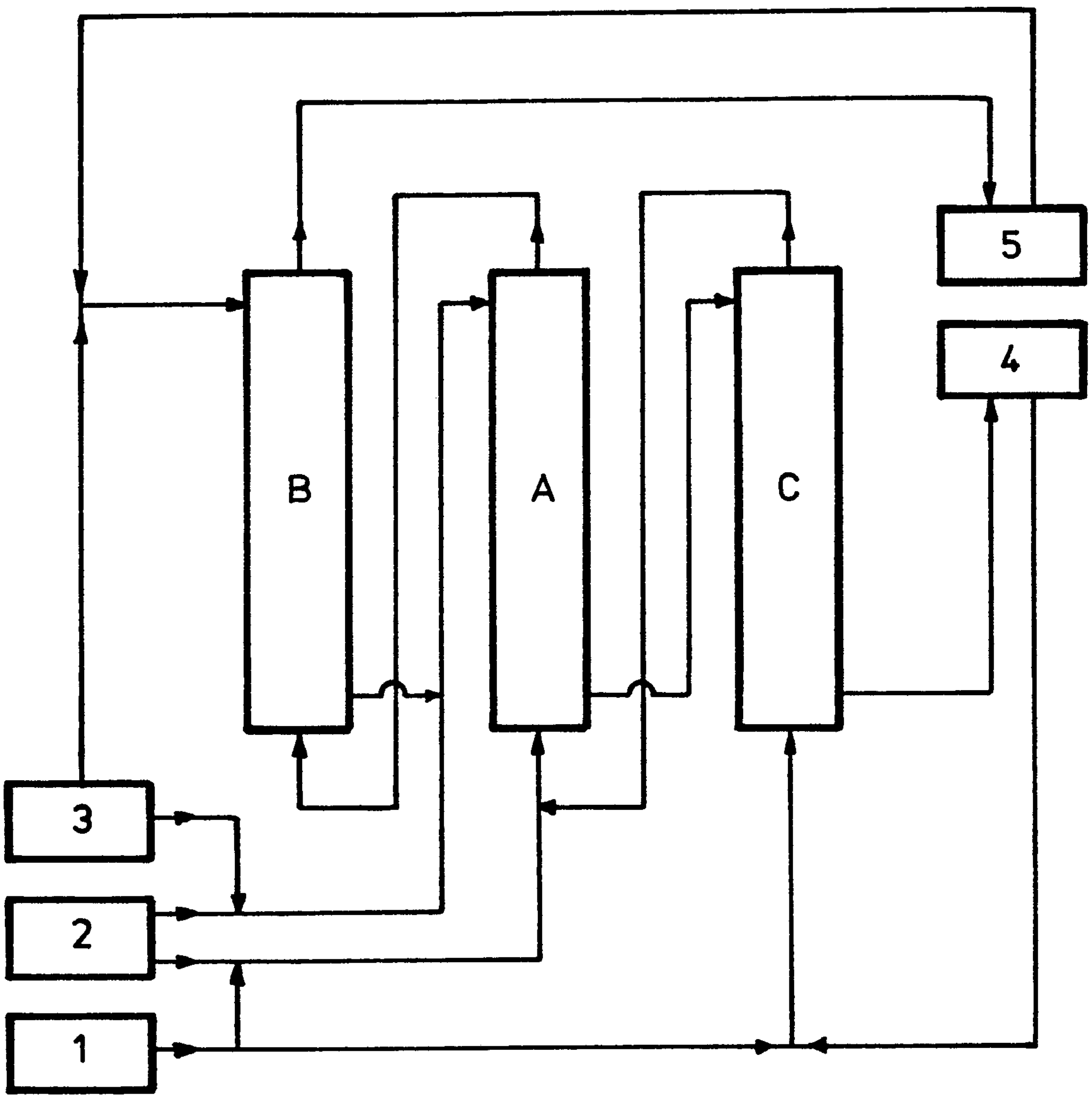


FIG.1